# EUROPEAN PATENT APPLICATION

(11) **EP 4 621 261 A1**
(43) Date of publication of application: **24.09.2025**
(21) Application number: 23907108.7
(22) Date of filing: 20.12.2023
(51) Int. Cl.: F16H 3/089, F16H 3/06

(54) **COUPLING DEVICE**

(30) Priority: 21.12.2022 JP 2022204448
(71) Applicant: HONDA MOTOR CO., LTD., Tokyo 105-8404 (JP)
(72) Inventor: SHIMADA, Kei, Wako-shi, Saitama 351-0193 (JP); ONO, Hiromi, Wako-shi, Saitama 351-0193 (JP); HARA, Hiroaki, Wako-shi, Saitama 351-0193 (JP); NAKAMURA, Kimiaki, Wako-shi, Saitama 351-0193 (JP); KANAI, Norishige, Wako-shi, Saitama 351-0193 (JP)
(74) Representative: Kiwit, Benedikt
(86) International application number: PCT/JP2023/045795
(87) International publication number: WO 2024/135748

(57) **Abstract**

An electric prosthetic leg (1) includes a below-knee member (110), an above-knee member (120), a knee joint mechanism (130) configured to couple the below-knee member (110) and the above-knee member (120) such that a formed angle formed therebetween is variable, and an enlarging and reducing device (200) capable of enlarging and reducing the formed angle. The enlarging and reducing device (200) includes a motor (M) and a transmission (T). The transmission (T) includes a first transmission mechanism (T1) configured to transmit power of the motor (M) at a first transmission ratio, and a second transmission mechanism (T2) configured to transmit the power of the motor (M) at a second transmission ratio different from the first transmission ratio. The enlarging and reducing device (200) includes a first connection and disconnection mechanism (210) configured to switch between connection and disconnection of power in the first transmission mechanism (T1), and a second connection and disconnection mechanism (220) configured to switch between connection and disconnection of power in the second transmission mechanism (T2).

## Description

### TECHNICAL FIELD

The present invention relates to a joint device.

### BACKGROUND ART

In the related art, as a joint device used for a coupling unit coupling two members, there is a joint device including an expansion-contraction device capable of varying a formed angle formed between the two members. An example of such a joint device is a prosthetic leg used for a knee joint. Patent Literature 1 discloses that a sensor for detecting contraction motion of a muscle at a cut-off end portion of an amputated leg is provided in a thigh socket of a prosthetic leg attached to the cut-off end portion of the amputated leg, and a throttling condition of a variable valve of a hydraulic cylinder for adjusting resistance of bending and stretching of a knee joint unit is controlled based on detection information from the sensor.

### CITATION LIST

### PATENT LITERATURE

Patent Literature 1: JPH11-019105A

### SUMMARY OF INVENTION

### TECHNICAL PROBLEM

However, in the prosthetic leg described in Patent Literature 1, although the resistance of bending and stretching can be generated, power of bending and stretching cannot be generated. Particularly, in order to smoothly ascend steps, it is necessary to stretch the knee joint in a state where a load is applied.

The present invention provides a joint device capable of stretching and bending a coupling unit with power of a power source.

### SOLUTION TO PROBLEM

The present invention is a joint device including:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission unit configured to transmit power of the power source,
the power transmission unit has:
   a first power transmission path for transmitting the power at a first transmission ratio; and
   a second power transmission path for transmitting the power at a second transmission ratio different from the first transmission ratio, and
the enlarging and reducing device includes:
   a first connection and disconnection mechanism configured to switch between connection and disconnection of power in the first power transmission path; and
   a second connection and disconnection mechanism configured to switch between connection and disconnection of power in the second power transmission path.

### ADVANTAGEOUS EFFECTS OF INVENTION

According to the present invention, the coupling unit can be stretched and bent via the power transmission unit which transmits the power of the power source.

### BRIEF DESCRIPTION OF DRAWINGS

[FIG. 1] FIG. 1 is a perspective view of an electric prosthetic leg 1 according to a first embodiment of the present invention as viewed obliquely from the rear.
[FIG. 2] FIG. 2 is a side view of the electric prosthetic leg 1 of FIG. 1.
[FIG. 3] FIG. 3 is a perspective view of an enlarging and reducing device 200 of the first embodiment that can be mounted on the electric prosthetic leg 1 of FIG. 1.
[FIG. 4] FIG. 4 is a cross-sectional view of the enlarging and reducing device 200 of FIG. 3.
[FIG. 5] FIG. 5 is a cross-sectional perspective view of a knee joint mechanism 130 of the electric prosthetic leg 1 in which the enlarging and reducing device 200 of FIG. 3 is assembled.
[FIG. 6] FIG. 6 is a cross-sectional view of a two-way clutch 280.
[FIG. 7] FIG. 7 is a perspective view of a retainer 282.
[FIG. 8] FIG. 8 is a view showing operations of an operation mechanism 240, in which (A) of FIG. 8 shows a state where a connection and disconnection unit 212 and a connection and disconnection unit 222 are in an off state, and (B) of FIG. 8 shows a state where the connection and disconnection unit 212 is in the off state and the connection and disconnection unit 222 is in an on state, and (C) of FIG. 8 shows a state where the connection and disconnection unit 212 is in the on state and the connection and disconnection unit 222 is in the off state.
[FIG. 9] (A) of FIG. 9 is a cross-sectional view showing a state where the connection and disconnection unit 222 is in the off state, and (B) of FIG. 9 is a view showing a position of an operation rod 241 at that time.
[FIG. 10] (A) of FIG. 10 is a cross-sectional view showing a state where the connection and disconnection unit 222 is operated from the off state to the on state, and (B) of FIG. 10 is a view showing a position of the operation rod 241 at that time.
[FIG. 11] (A) of FIG. 11 is a cross-sectional view showing a normal-rotation on state of the connection and disconnection unit 222, and (B) of FIG. 11 is a view showing a position of the operation rod 241 at that time.
[FIG. 12] (A) of FIG. 12 is a cross-sectional view showing a reverse-rotation on state of the connection and disconnection unit 222, and (B) of FIG. 12 is a view showing a position of the operation rod 241 at that time.
[FIG. 13] (A) of FIG. 13 is a cross-sectional view showing a state where the connection and disconnection unit 222 is operated from the on state to the off state, and (B) of FIG. 13 is a view showing a position of the operation rod 241 at that time.
[FIG. 14] FIG. 14 is a perspective view of an enlarging and reducing device 200A of a second embodiment that can be mounted on the electric prosthetic leg 1 of FIG. 1.
[FIG. 15] FIG. 15 is a cross-sectional view of the enlarging and reducing device 200A of FIG. 14.
[FIG. 16] FIG. 16 is a cross-sectional perspective view of the knee joint mechanism 130 of the electric prosthetic leg 1 in which the enlarging and reducing device 200A of the second embodiment is assembled.
[FIG. 17] FIG. 17 is a perspective view of an enlarging and reducing device 200B of a third embodiment that can be mounted on the electric prosthetic leg 1 of FIG. 1.
[FIG. 18] FIG. 18 is a cross-sectional view of the enlarging and reducing device 200B of FIG. 17.
[FIG. 19] FIG. 19 is an enlarged view of a planetary gear mechanism 260 in the enlarging and reducing device 200B of FIG. 18.
[FIG. 20] FIG. 20 is a B-B cross-sectional view of FIG. 19.
[FIG. 21] FIG. 21 is a diagram showing actions (stair ascending actions) of a user and an electric prosthetic leg when ascending stairs.
[FIG. 22] FIG. 22 is a diagram showing actions (flat ground walking actions) of the user and the electric prosthetic leg when walking on a flat ground.

### DESCRIPTION OF EMBODIMENTS

Hereinafter, an electric prosthetic leg as an embodiment of a joint device of the present invention will be described below with reference to the drawings. In the following description, a front-rear direction, a left-right direction, and an upper-lower direction are defined with reference to a user of the electric prosthetic leg. In the drawings, a front side of the electric prosthetic leg is denoted by Fr, a rear side is denoted by Rr, a left side is denoted by L, a right side is denoted by R, an upper side is denoted by U, and a lower side is denoted by D.

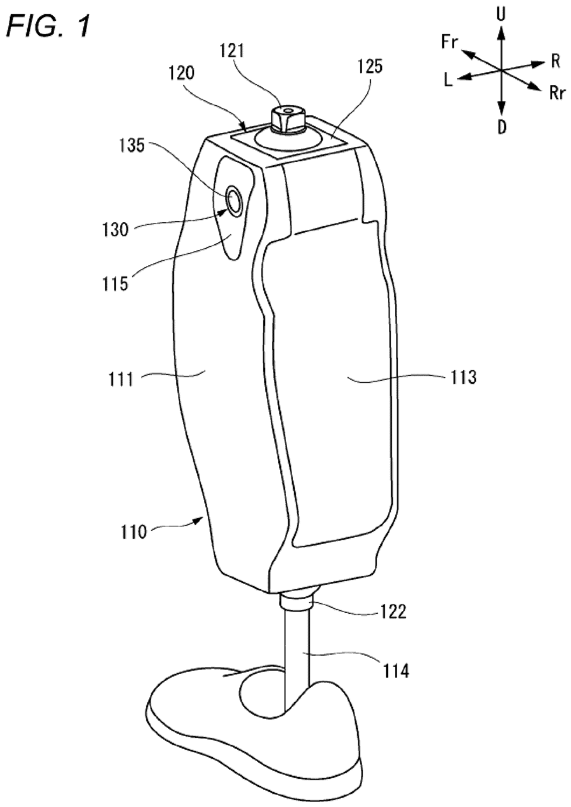

As shown in FIG. 1, an electric prosthetic leg 1 according to the present embodiment is a prosthetic leg that is attached to a leg of a person who does not have a knee. The electric prosthetic leg 1 includes: a below-knee member 110 positioned on a lower side of the knee, an above-knee member 120 positioned on an upper side of the knee and attached to a thigh, a knee joint mechanism 130 that couples the below-knee member 110 and the above-knee member 120 such that a formed angle formed therebetween is variable, an enlarging and reducing device 200 capable of enlarging and reducing the formed angle formed between the below-knee member 110 and the above-knee member 120, and a battery B that supplies power to the enlarging and reducing device 200 and the like.

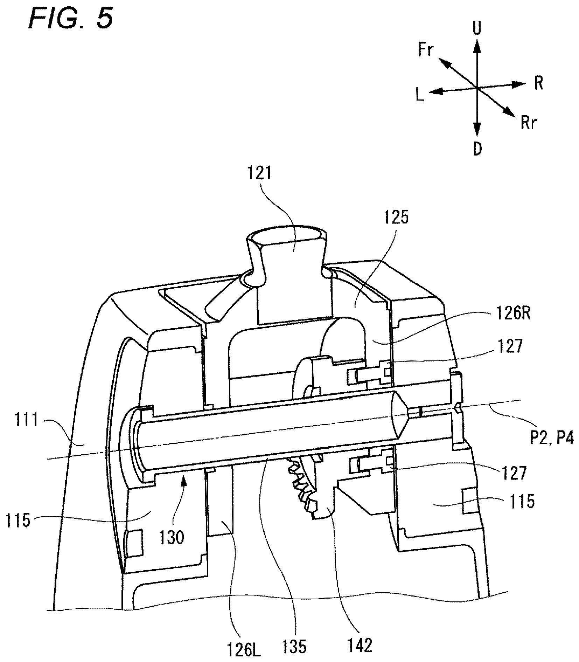

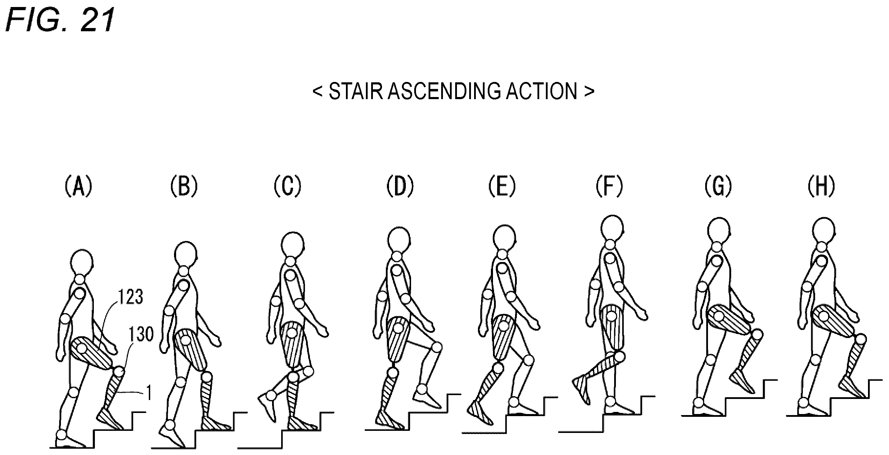

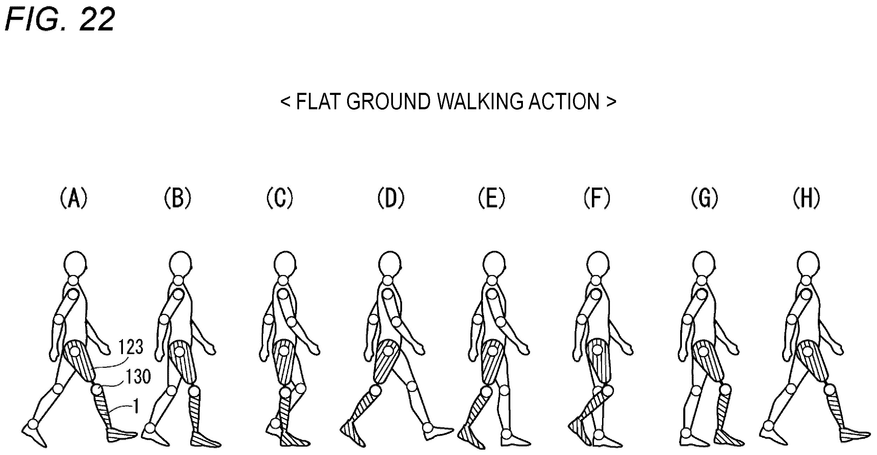

Referring also to FIG. 5, above-knee member 120 includes adapter 121 coupled to a socket (not shown), upper wall portion 125 to which the adapter 121 is attached, and a pair of left and right side wall portions 126L and 126R coupled to upper wall portion 125. The socket is a joint member provided in the thigh 123 (see FIGS. 21 and 22), and the above-knee member 120 is integrated with the thigh 123 by coupling the adapter 121 to the socket.

The below-knee member 110 includes a box-shaped main frame 111 having an open rear portion, a detachable rear cover 113 that covers the rear opening of the main frame 111 in an openable and closable manner, and an adapter 122 attached to a lower surface of the main frame 111.

The above-knee member 120 is provided on an upper portion of the main frame 111 of the below-knee member 110 via a coupling axis 135 that constitutes the knee joint mechanism 130, and a leg 114 extending downward is coupled to the adapter 122 of the main frame 111.

An enlarging and reducing device 200 capable of enlarging and reducing the formed angle formed between the below-knee member 110 and the above-knee member 120 is provided in a space formed by the below-knee member 120 and the above-knee member 110. The enlarging and reducing device 200 is configured to enlarge and reduce the formed angle formed between the below-knee member 110 and the above-knee member 120 by meshing of gears.

### (First Embodiment)

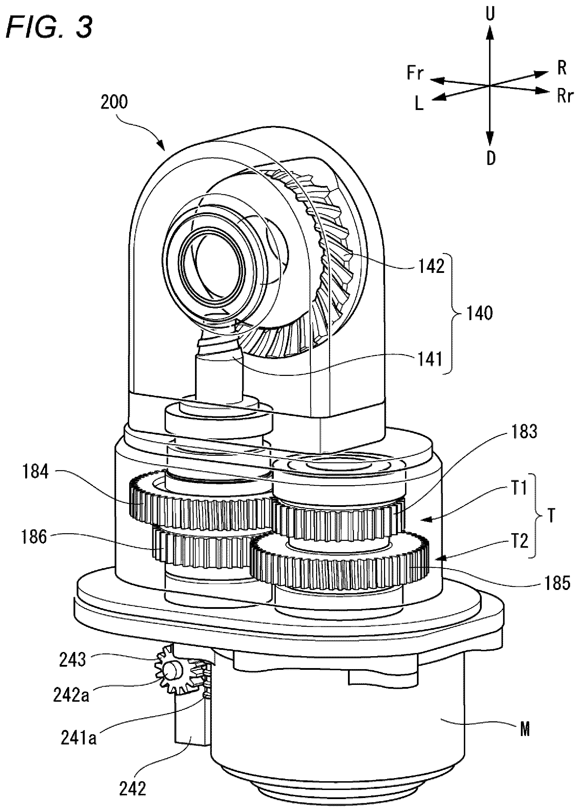

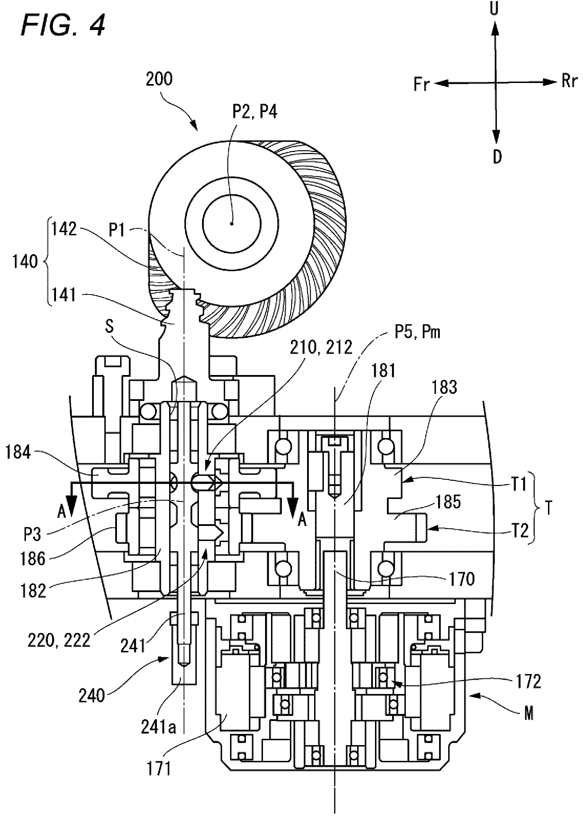

As shown in FIGS. 3 and 4, the enlarging and reducing device 200 includes a motor M that outputs rotational power, a transmission T that transmits the power of the motor M, a bevel gear mechanism 140 that can enlarge and reduce the formed angle formed between the below-knee member 110 and the above-knee member 120, and a first connection and disconnection mechanism 210 and a second connection and disconnection mechanism 220 which are provided in the transmission T.

The motor M is, for example, a permanent-magnet electric motor, and is disposed on a rear side of the transmission T and below the transmission T. The motor M is a motor with a built-in gear mechanism, including a motor body 171, a gear mechanism unit 172 that decelerates output rotation of the motor body 171, and an output shaft 170 that has a rotation axis Pm.

The bevel gear mechanism 140 includes a first bevel gear 141 that is disposed on a side opposite to the motor M with respect to the transmission T on a power transmission path of the motor M and is supported by the below-knee member 110, and a second bevel gear 142 that meshes with the first bevel gear 141 to be capable of transmitting rotation therebetween and is supported by the above-knee member 120. The first bevel gear 141 and the second bevel gear 142 constitute the bevel gear mechanism 140 in which teeth are formed on a conical or truncated conical outer surface.

A rotation axis P1 of the first bevel gear 141 and a rotation axis P2 of the second bevel gear 142 are arranged to extend in directions orthogonal to each other. That is, the first bevel gear 141 and the second bevel gear 142 are configured to transmit the rotation therebetween using a conical gear meshing mechanism. In the present embodiment, the rotation is transmitted by the meshing of two gears, but the rotation may be transmitted by friction between two rollers. That is, the expression "capable of transmitting rotation" may refer to transmission based on the meshing of gears, transmission based on the friction between rollers, or the like. The rotation axis P1 of the first bevel gear 141 and the rotation axis P2 of the second bevel gear 142 may be on the same plane or may not be on the same plane. As in the present embodiment, the bevel gear mechanism 140 in which the two rotation axes P1 and P2 are not on the same plane is referred to as a hypoid gear (registered trademark). The rotation axis P1 of the first bevel gear 141 and a rotation axis P3 of a second shaft 182 to be described later are on the same line. The rotation axis P2 of the second bevel gear 142 and an axis P4 of the coupling axis 135 are on the same line.

In relation to the rotation axis Pm of the motor M, the rotation axis P1 of the first bevel gear 141 (the second shaft 182) is arranged to extend in a direction parallel to the rotation axis Pm of the motor M, and the rotation axis P2 of the rotation axis P2 of the second bevel gear 142 is arranged to extend in a direction orthogonal (perpendicular) to the rotation axis Pm of the motor M. The motor M is disposed at a position overlapping at least a part of the second bevel gear 142 as viewed in the upper-lower direction orthogonal to the rotation axis P2 of the second bevel gear 142.

The first bevel gear 141 is configured to integrally rotate with the second shaft 182 which is an output element of the transmission T to be described later. The first bevel gear 141 transmits, to the second bevel gear 142, the power of the motor M transmitted via the transmission T.

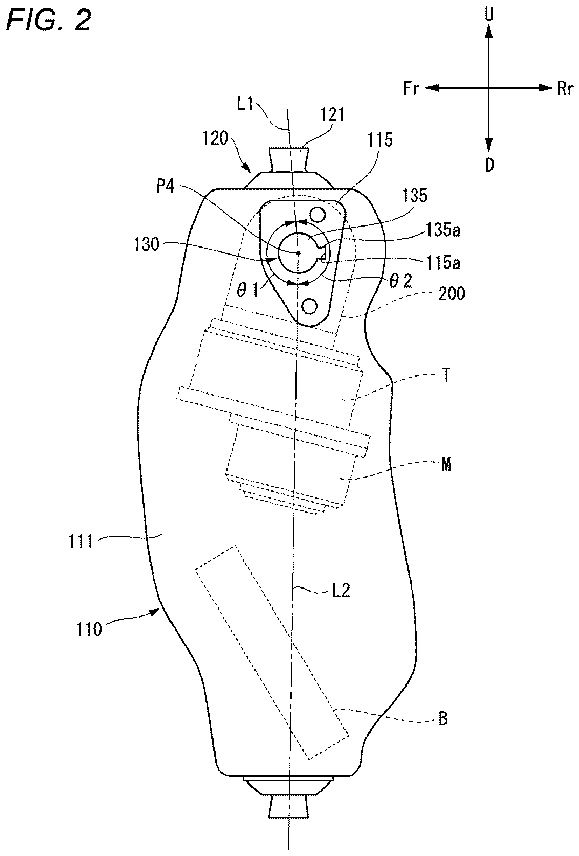

The second bevel gear 142 is provided to be rotatable relative to the coupling axis 135 and rotatable integrally with the above-knee member 120. More specifically, as shown in FIG. 2, the coupling axis 135 is prevented from rotating by fitting a protrusion portion 135a of the coupling axis 135 into a recess portion 115a provided in a through hole through which the coupling axis 135 of the coupling axis support plate 115 of the main frame 111 penetrates. As shown in FIG. 5, the second bevel gear 142 is integrally fastened to the right side wall portion 126R of the above-knee member 120 by bolts 127. The side wall portions 126L and 126R are configured to rotate relative to the coupling axis 135 via bearings (not shown). Accordingly, when the second bevel gear 142 meshing with the first bevel gear 141 rotates, the above-knee member 120 rotates about the coupling axis 135. Therefore, the formed angle formed between the above-knee member 120 and the below-knee member 110 varies.

Here, as shown in FIG. 2, the formed angle formed between the above-knee member 120 and the below-knee member 110 is an angle defined by a first virtual line L1 connecting the axis P4 of the coupling axis 135 of the knee joint mechanism 130 and the adapter 121 of the above-knee member 120, and a second virtual line L2 extending downward in a vertical direction through the axis P4 of the coupling axis 135 of the knee joint mechanism 130 and the below-knee member 110. Among the formed angles formed between the below-knee member 110 and the above-knee member 120 with the coupling axis 135 of the knee joint mechanism 130 as a center, an angle on one side in one circumference is defined as a first formed angle θ1, and an angle on the other side in the circumference is defined as a second formed angle θ2. When a smaller minimum angle, of the first formed angle θ1 and the second formed angle θ2, formed in a range of relative movement between the below-knee member 110 and the above-knee member 120 is the second formed angle θ2, a formed angle (knee-back angle) formed on a back side of the knee of the user of the electric prosthetic leg 1 is defined as the second formed angle θ2. The first formed angle θ1 takes a value of about 175 [deg] to 300 [deg], and the second formed angle θ2 takes a value of about 60 [deg] to 185 [deg].

FIG. 2 shows a stretched state of the knee joint mechanism 130, in which the first formed angle θ1 is about 175 [deg], and the second formed angle θ2 is about 185 [deg]. In the second bevel gear 142, the teeth may be formed over an entire circumference, but in the present embodiment, the teeth are only formed partially in the circumferential direction to satisfy a movable range of the second formed angle θ2.

Returning to FIGS. 3 and 4, the transmission T includes a first transmission mechanism T1 that transmits the power of the motor M to the first bevel gear 141 at a first transmission ratio, and a second transmission mechanism T2 that transmits the power of the motor M to the first bevel gear 141 at a second transmission ratio, which is different from the first transmission ratio. The first transmission mechanism T1 and the second transmission mechanism T2 are switched between a power non-transmittable state and a power transmittable state by switching a disconnection state and a connection state of each of the connection and disconnection mechanisms 210 and 220.

According to such a transmission T, by providing two power transmission paths with different transmission ratios, an operating speed and generated power for stretching and bending in the knee joint mechanism 130 can be switched. As long as the first transmission ratio is different from the second transmission ratio, one of the first transmission mechanism T1 and the second transmission mechanism T2 may be a speed reduction mechanism and the other may be a speed increasing mechanism, or one may be a constant speed mechanism and the other may be a speed reduction mechanism or a speed increasing mechanism, or both may be speed reduction mechanisms, or both may be speed increasing mechanisms.

The first transmission ratio is a ratio of a post-transmission rotation speed, which is a rotation speed on a motor M opposite-side (a first bevel gear 141 side) in the first transmission mechanism T1, with respect to a pre-transmission rotation speed, which is a rotation speed on a motor M side in the first transmission mechanism T1. The second transmission ratio is a ratio of a post-transmission rotation speed, which is a rotation speed on a motor M opposite-side (a first bevel gear 141 side) in the second transmission mechanism T2, with respect to a pre-transmission rotation speed, which is a rotation speed on a motor M side in the second transmission mechanism T2.

For example, when the first transmission ratio of the first transmission mechanism T1 is smaller than 1, the rotation speed on the motor M opposite-side (the first bevel gear 141 side) becomes lower than the rotation speed on the motor M side, and a torque increases. When the second transmission ratio of the second transmission mechanism T2 is larger than 1, the rotation speed on the motor M opposite-side (the first bevel gear 141 side) becomes higher than the rotation speed on the motor M side, and a torque decreases. In the present embodiment, the first transmission ratio is set to be smaller than 1 and the second transmission ratio is set to be larger than 1, and the first transmission mechanism T1 is disposed above the second transmission mechanism T2.

The first transmission mechanism T1 and the second transmission mechanism T2 include a first shaft 181 coupled to the output shaft 170 to be integrally rotatable on an upward extension line of the output shaft 170 of the motor M, and a second shaft 182 coupled to the first bevel gear 141 to be integrally rotatable on a downward extension line of the first bevel gear 141. In a power flow in which the power of the motor M flows to the bevel gear mechanism 140 via the transmission T, the first shaft 181 is an input element of the transmission T, and the second shaft 182 is an output element of the transmission T.

The first transmission mechanism T1 includes a first drive gear 183 and a first driven gear 184 that mesh with each other. The first drive gear 183 is supported by the first shaft 181 to be integrally rotatable, and the first driven gear 184 is supported by the second shaft 182 to be relatively rotatable. The first drive gear 183 and the first shaft 181 have the same rotation axis P5, and the first driven gear 184 and the second shaft 182 have the same rotation axis P3. The first transmission mechanism T1 of the present embodiment is a deceleration transmission mechanism in which the first drive gear 183 has a diameter smaller than that of the first driven gear 184, and can cause the first bevel gear 141 to expand and contract at low speed and high torque.

The second transmission mechanism T2 includes a second drive gear 185 and a second driven gear 186 that mesh with each other. The second drive gear 185 is supported by the first shaft 181 to be integrally rotatable, and the second driven gear 186 is supported by the second shaft 182 to be relatively rotatable. The second drive gear 185 and the first shaft 181 have the same rotation axis P5, and the second driven gear 186 and the second shaft 182 have the same rotation axis P3. The second transmission mechanism T2 of the present embodiment is an acceleration transmission mechanism in which the second drive gear 185 has a diameter larger than that of the second driven gear 186, and can cause the first bevel gear 141 to expand and contract at high speed and low torque. In the present embodiment, the second transmission mechanism T2 is disposed below the first transmission mechanism T1, but the second transmission mechanism T2 may be disposed above the first transmission mechanism T1.

In the present embodiment, the first drive gear 183 and the second drive gear 185, which are supported by the first shaft 181 to be integrally rotatable, are integrally formed so as to be integrally rotatable. Further, as described above, the rotation axis P3 of the first driven gear 184 and the second driven gear 186, which have the same rotation axis as the second shaft 182, and the rotation axis P1 of the first bevel gear 141 are on the same line, and the first bevel gear 141, the first driven gear 184, and the second driven gear 186 are disposed in this order from above. Further, the rotation axis P5 of the first drive gear 183 and the second drive gear 185, which have the same rotation axis as the first shaft 181, and the rotation axis Pm of the motor M are on the same line, and the first drive gear 183, the second drive gear 185, and the motor M are disposed in this order from above.

The first connection and disconnection mechanism 210 includes a connection and disconnection unit 212 provided between the first driven gear 184 and the second shaft 182. The second connection and disconnection mechanism 220 includes a connection and disconnection unit 222 provided between the second driven gear 186 and the second shaft 182. These connection and disconnection units 212 and 222 are disposed on the rotation axis P3 of the second shaft 182. The connection and disconnection units 212 and 222 have a common configuration and are configured to be switched between a disconnection state where the power transmission path is disconnected and a connection state where the power transmission path is connected. Details of the connection and disconnection units 212 and 222 will be described later.

As shown in FIGS. 2 and 3, the enlarging and reducing device 200 is unitized and accommodated in an internal space of the main frame 111. The enlarging and reducing device 200 is fixed to the main frame 111 by a bracket (not shown) except for the second bevel gear 142. In the internal space of the main frame 111, the battery B is fixed below the enlarging and reducing device 200.

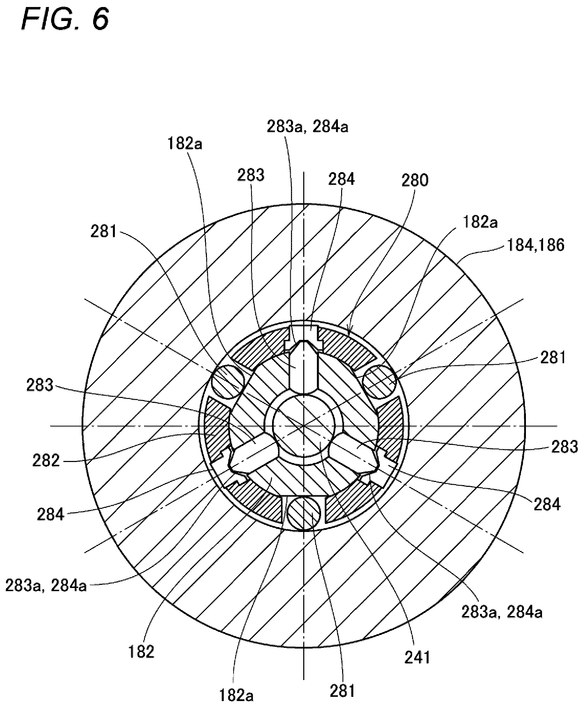

Next, the details of the connection and disconnection units 212 and 222 will be described with reference to FIG. 6 and subsequent drawings.

The connection and disconnection units 212 and 222 have a common configuration and are configured to be switched between the disconnection state where the power transmission path is disconnected and the connection state where the power transmission path is connected. Each of the connection and disconnection units 212 and 222 of the present embodiment is configured using a two-way clutch 280 with a forced free function, as shown in FIG. 6. The two-way clutch 280 includes a plurality of (three in the present embodiments) rollers 281 that are disposed between an outer peripheral surface portion of the second shaft 182 and inner peripheral surface portions of the gears 184 and 186, a retainer 282 that holds the plurality of rollers 281 at predetermined intervals, an operation mechanism 240, a plurality of (three in the present embodiment) pins 283 that penetrate the second shaft 182 in a radial direction and are operated by the operation mechanism 240 to a forced free position and a forced free release position, and a plurality of (three in the present embodiment) guides 284 that are provided in the retainer 282 and defines a relative rotational position of the retainer 282 with respect to the second shaft 182 when the pins 283 are in the forced free position. The rollers 281 may be balls or sprags.

A distance A in the radial direction between the outer peripheral surface portion of the second shaft 182 and the inner peripheral surface portions of the gears 184 and 186 is smaller than a diameter B of each of the rollers 281. Further, flat portions 182a are formed on an outer peripheral portion of the second shaft 182 at predetermined intervals in a circumferential direction, and on a center side in the circumferential direction of each of the flat portions 182a, the distance A is larger than the diameter B.

In other words, when the rollers 281 are held at center portions of the respective flat portions 182a in the circumferential direction, the rollers 281 do not mesh with the outer peripheral surface portion of the second shaft 182 and the inner peripheral surface portions of the gears 184 and 186 (non-engagement state), and relative rotation between the second shaft 182 and the gears 184 and 186 is allowed (forced free state).

On the other hand, when the rollers 281 are allowed to move in the circumferential direction relative to the second shaft 182, the rollers 281 mesh with the outer peripheral surface portion of the second shaft 182 and the inner peripheral surface portions of the gears 184 and 186 (engagement state), and the second shaft 182 and the gears 184 and 186 are connected to be rotatable integrally in two directions (forced free release state).

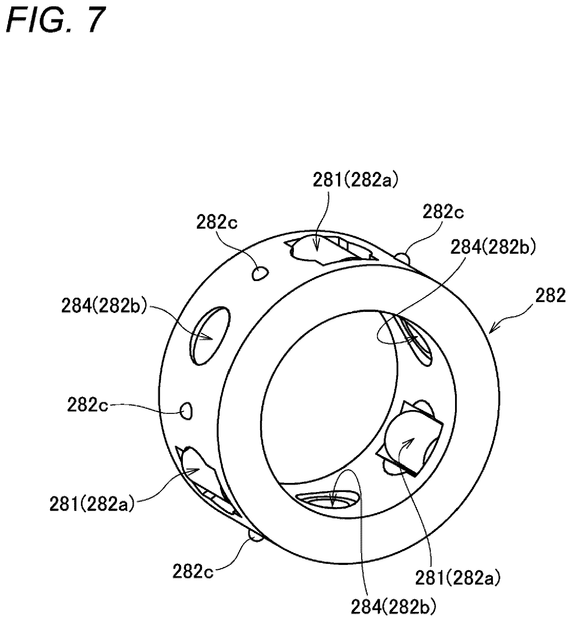

As shown in FIG. 7, the retainer 282 includes a plurality of roller holding portions 282a that are ring-shaped and capable of relatively rotating with respect to the second shaft 182 and the gears 184 and 186, and that hold the rollers 281, and a plurality of guide holding portions 282b that hold the guides 284.

**In** an outer peripheral surface of the retainer 282, a plurality of rubber balls 282c are embedded at predetermined intervals in the circumferential direction. These rubber balls 282c prevent unintended idling in the forced free release state by generating moderate friction between the gears 184 and 186 and the retainer 282. The members for generating friction between the gears 184 and 186 and the retainer 282 are not limited to the rubber balls 282c, and may be O-rings.

Returning to FIG. 6, each of the pins 283 includes a conical protrusion portion 283a on an outer end in the radial direction, and each of the guides 284 includes, on an inner end surface thereof in the radial direction, a conical recess portion 284a that fits to (engages with) the protrusion portion 283a. When the protrusion portion 283a of the pin 283 fits to the recess portion 284a of the guide 284, a relative rotation position of the retainer 282 with respect to the second shaft 182 is positioned at a predetermined position where the retainer 282 is in the forced free state by a guiding effect of the pins 283 and the guides 284.

The operation mechanism 240 includes an operation rod 241 configured to intermittently operate the connection and disconnection units 212 and 222, and a servo motor 242 (see FIG. 3) that linearly moves the operation rod 241. The servo motor 242 is disposed adjacent to the motor M. That is, the servo motor 242 is disposed on the same side as the motor M with respect to the transmission T. The servo motor 242 and the motor M may be unitized.

The second shaft 182 is a hollow shaft having an internal space S extending in a rotation axis direction (also referred to as the upper-lower direction), and the operation rod 241 is disposed in the internal space S. The operation rod 241 is provided with a rack 241a at a lower end exposed from the internal space S. As shown in FIG. 3, a pinion 243 provided on an output shaft 242a of the servo motor 242 meshes with the rack 241a, and a position of the operation rod 241 in the upper-lower direction is switched according to the drive of the servo motor 242.

Small-diameter portions 241b1 and 241b2 and large-diameter portions 241c1 to 241c3, which will be described later, are formed on an outer peripheral portion of the operation rod 241, and abut on inner-diameter end portions of the pins 283. When the small-diameter portions 241b1 and 241b2 and the large-diameter portions 241c1 to 241c3 move the pins 283 forward and backward in the radial direction of the second shaft 182 in response to a position of the operation rod 241, the states of the connection and disconnection units 212 and 222 are switched.

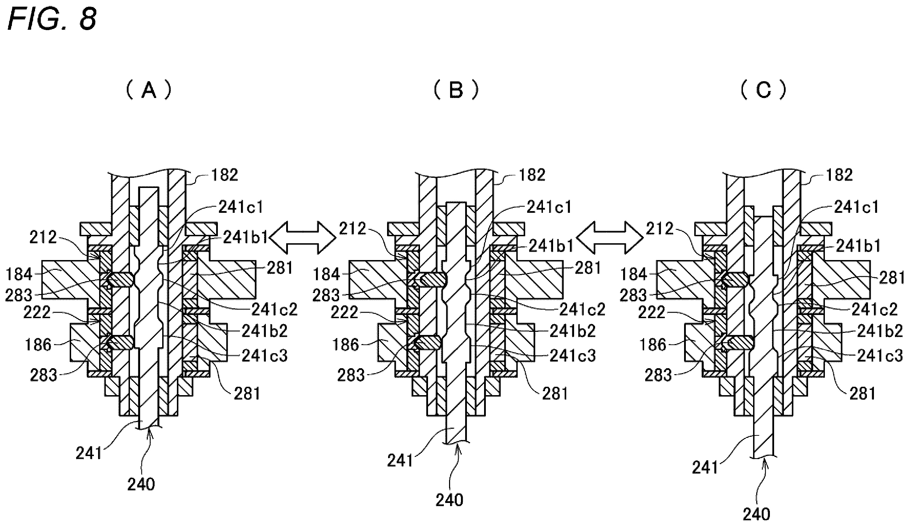

More specifically, as shown in FIG. 8, on the outer peripheral portion of the operation rod 241, in the order from the upper side, the first large-diameter portion 241c1, the first small-diameter portion 241b1, the second large-diameter portion 241c2, the second small-diameter portion 241b2, and the third large-diameter portion 241c3 formed with predetermined lengths and intervals therebetween. The operation rod 241 is provided to be capable of simultaneously controlling the two connection and disconnection units 212 and 222, but may be provided separately for each of the connection and disconnection units 212 and 222.

In the following description, operation of the operation mechanism 240 that simultaneously controls the connection and disconnection units 212 and 222 will be described with reference to FIG. 8.

As shown in FIG. 8, the connection and disconnection units 212 and 222 are switched between the forced free state (hereinafter referred to as an off state as appropriate) and the forced free release state (hereinafter referred to as an on state as appropriate) by the operation mechanism 240.

When the operation rod 241 of the operation mechanism 240 is in an upper position shown in (A) of FIG. 8, the third large-diameter portion 241c3 pushes out the pin 283 of the connection and disconnection unit 222 in an outer diameter direction while the second large-diameter portion 241c2 pushes out the pin 283 of the connection and disconnection unit 212 in the outer diameter direction, thus setting the connection and disconnection unit 222 and the connection and disconnection unit 212 to the off state.

Further, when the operation rod 241 of the operation mechanism 240 is in a middle position shown in (B) of FIG. 8, the third large-diameter portion 241c3 pushes out the pin 283 of the connection and disconnection unit 222 in the outer diameter direction while the first small-diameter portion 241b1 allows the pin 283 of the connection and disconnection unit 212 to return in an inner diameter direction, thereby setting the connection and disconnection unit 212 to the on state and the connection and disconnection unit 222 to the off state.

Further, when the operation rod 241 of the operation mechanism 240 is in a lower position shown in (C) of FIG. 8, the second small-diameter portion 241b2 allows the pin 283 of the connection and disconnection unit 222 to return in the inner diameter direction while the first large-diameter portion 241c1 pushes out the pin 283 of the connection and disconnection unit 212 in the outer diameter direction, thus setting the connection and disconnection unit 212 to the off state and the connection and disconnection unit 222 to the on state.

That is, in the two-way clutch 280 constituting the connection and disconnection units 212 and 222, the roller 281 is an engagement element, and the operation rod 241, the pin 283, the guide 284, and the retainer 282 correspond to an operation unit that operates the engagement element (the roller 281) between the engagement state and the non-engagement state. Further, among the elements constituting the operation unit, the pin 283, the guide 284, and the retainer 282 correspond to an actuation element that moves the engagement element (the roller 281), and the operation rod 241 corresponds to an operation element provided to be able to operate the actuation element. Further, among the elements constituting the actuation element, the pin 283 is an advance and retraction element provided to be capable of advancing and retracting along the radial direction, and the guide 284 and the retainer 282 correspond to a holder that holds the advance and retraction element (the pin 283) to be capable of advancing and retracting.

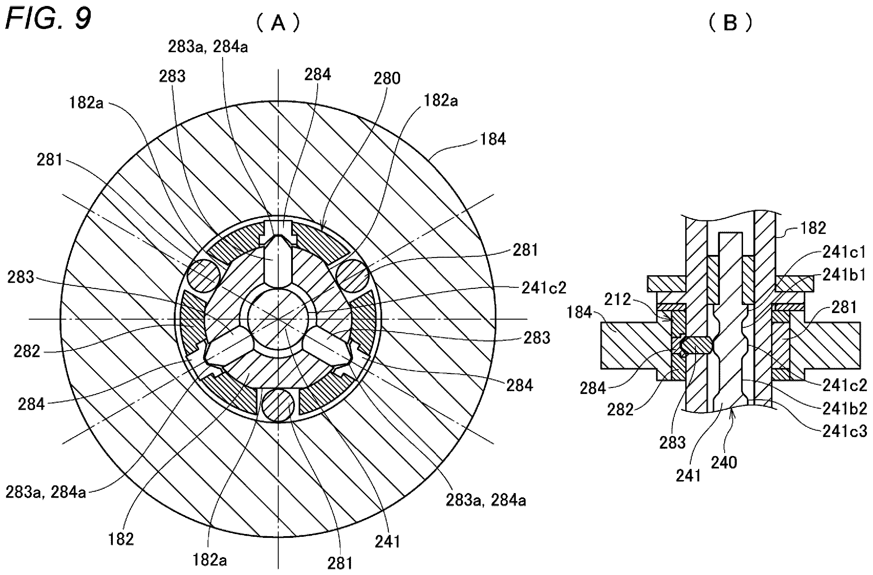

Next, the operation of the two-way clutch 280 will be described with reference to FIGS. 9 to 13, taking the connection and disconnection unit 212 as an example. In the following example, a case of a transition from (A) to (B) to (C) in FIG. 8 in the connection and disconnection unit 212 will be described as an example.

As shown in (A) and (B) of FIG. 9, in a state where the second large-diameter portion 241c2 of the operation rod 241 pushes out the pin 283 of the connection and disconnection unit 212 in the outer diameter direction, the protrusion portion 283a of the pin 283 fits to the recess portion 284a of the guide 284, and the relative rotational position of the retainer 282 with respect to the second shaft 182 is fixed at a predetermined position. In this state, since the rollers 281 are held at the center portions of the respective flat portions 182a in the circumferential direction, the rollers 281 do not mesh with the outer peripheral surface portion of the second shaft 182 and the inner peripheral surface portion of the first driven gear 184, and the state becomes the off state where the relative rotation between the second shaft 182 and the first driven gear 184 is allowed.

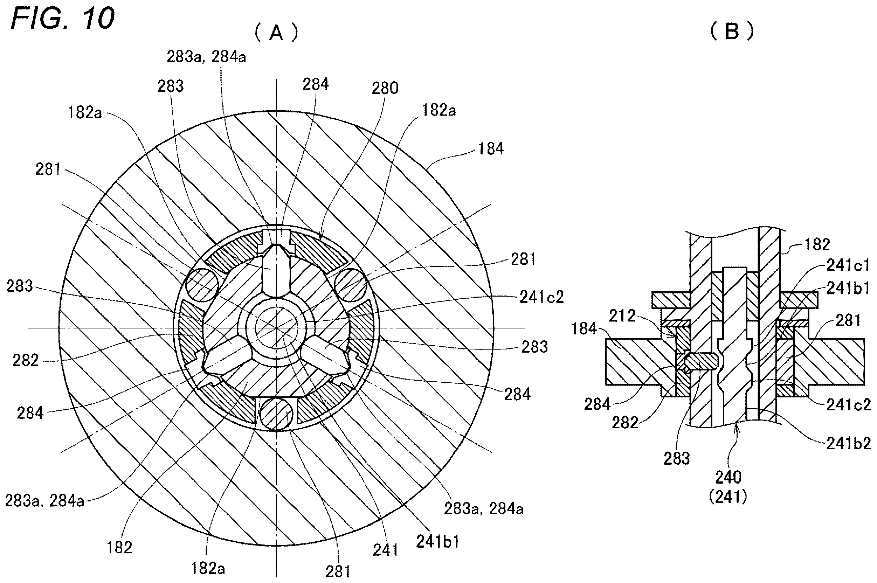

(A) and (B) of FIG. 10 show a state where the operation rod 241 moves from the position where the second large-diameter portion 241c2 pushes out the pin 283 of the connection and disconnection unit 212 in the outer diameter direction to the position where the first small-diameter portion 241b1 allows the pin 283 to return in the inner diameter direction.

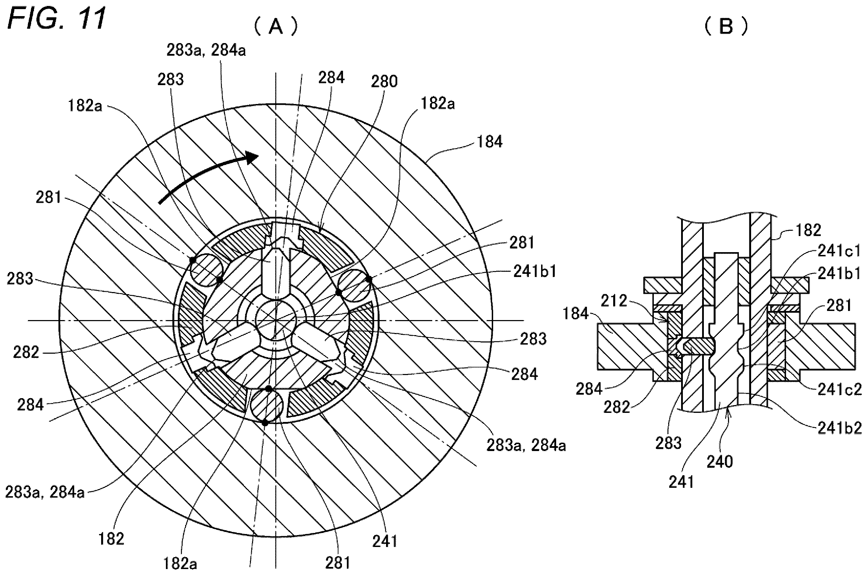

As shown in (A) and (B) of FIG. 11, in a state where the pin 283 is allowed to return in the inner diameter direction, when relative rotation occurs between the second shaft 182 and the first driven gear 184 in a normal rotation direction shown by an arrow in the drawing, the retainer 282 that rotates together with the first driven gear 184 moves the rollers 281 in the normal rotation direction with respect to the second shaft 182. Accordingly, the rollers 281 mesh with the outer peripheral surface portion of the second shaft 182 and the inner peripheral surface portion of the first driven gear 184, and a normal-rotation on state is created in which the second shaft 182 and the first driven gear 184 are rotated integrally in the normal rotation direction.

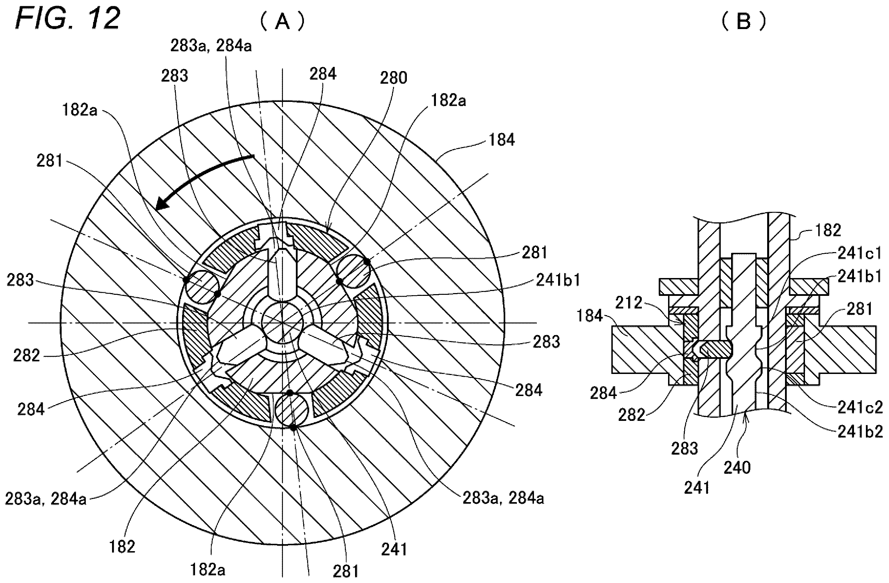

As shown in (A) and (B) of FIG. 12, in a state where the pin 283 is allowed to return in the inner diameter direction, when relative rotation occurs between the second shaft 182 and the first driven gear 184 in a reverse rotation direction shown by an arrow in the drawing, the retainer 282 that rotates together with the first driven gear 184 moves the rollers 281 in the reverse rotation direction with respect to the second shaft 182. Accordingly, the rollers 281 mesh with the outer peripheral surface portion of the second shaft 182 and the inner peripheral surface portion of the first driven gear 184, and a reverse-rotation on state is created in which the second shaft 182 and the first driven gear 184 are rotated integrally in the reverse rotation direction.

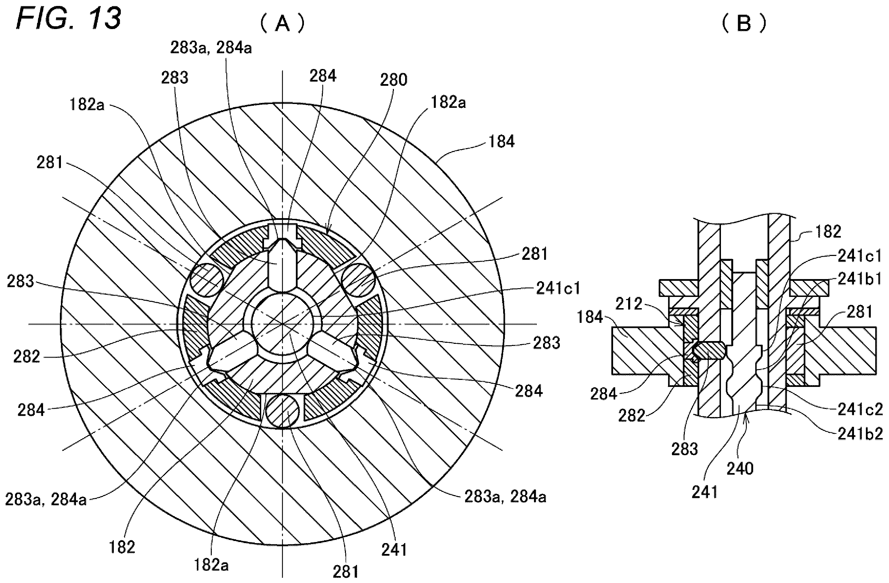

As shown in (A) and (B) of FIG. 13, when the operation rod 241 moves from a position where the first small-diameter portion 241b1 allows the pin 283 of the connection and disconnection unit 212 to return in the inner diameter direction, to a position where the first large-diameter portion 241c1 pushes out the pin 283 in the outer diameter direction, the protrusion portion 283a of the pin 283 fits to the recess portion 284a of the guide 284, and the relative rotation position of the retainer 282 with respect to the second shaft 182 is fixed at a predetermined position, by the guiding effect of the pin 283 and the guide 284. In this state, since the rollers 281 are held at the center portions of the respective flat portions 182a in the circumferential direction, the rollers 281 do not mesh with the outer peripheral surface portion of the second shaft 182 and the inner peripheral surface portion of the first driven gear 184, and the state becomes the off state where the relative rotation between the second shaft 182 and the first driven gear 184 is allowed.

In the above embodiment, the connection and disconnection units 212 and 222 are provided on a second shaft 182 side, but may be provided on a first shaft 181 side. That is, the connection and disconnection unit 212 of the first connection and disconnection mechanism 210 is provided between the first drive gear 183 and the first shaft 181, and the connection and disconnection unit 222 of the second connection and disconnection mechanism 220 is provided between the second drive gear 185 and the first shaft 181. Further, the connection and disconnection units 212 and 222 may be provided on both the second shaft 182 side and the first shaft 181 side.

In the electric prosthetic leg 1 configured in this way, it is possible to smoothly ascend stairs, which has been required to be done one by one by a leg on a non-prosthetic leg side, with a passive prosthetic leg including a passive damper in the related art. FIG. 21 is a diagram showing actions (stair ascending actions) of the user and the electric prosthetic leg 1 when ascending stairs. (A) to (D) of FIG. 21 are diagrams showing a stance phase, (D) and (E) of FIG. 21 are diagrams showing a transition phase from a stance to an initial swing, (E) to (G) of FIG. 21 are diagrams showing an initial swing phase, (G) of FIG. 21 is a diagram showing a transition phase from the initial swing to a terminal swing and a terminal swing phase, and (H) of FIG. 21 is a diagram showing a transition phase from the terminal swing to the stance and the stance phase.

Specifically, as shown in (A) to (D) of FIG. 21, large power is required when the knee joint mechanism 130 is stretched from a bending state, in a state where a load is applied to the electric prosthetic leg 1 when moving the electric prosthetic leg 1 forward to ascending stairs.

At this time, the transmission T is set to a first speed-change state where the operation rod 241 is located at the middle position ((B) of FIG. 8). In this speed-change state, the connection and disconnection unit 212 is in the on state and the connection and disconnection unit 222 is in the off state, and the motor M and the bevel gear mechanism 140 are in the power transmission state via the first transmission mechanism T1.

In this state, when the motor M is rotated in the normal rotation direction, the power of the motor M is transmitted to the first shaft 181, the first drive gear 183, the first driven gear 184, the connection and disconnection unit 212 of the first connection and disconnection mechanism 210, the second shaft 182, and the bevel gear mechanism 140. With the rotation of the first bevel gear 141 fixed to the below-knee member 110, the second bevel gear 142 fixed to the above-knee member 120 rotates, and thereby the above-knee member 120 rotates about the coupling axis 135 with respect to the below-knee member 110, and knee joint mechanism 130 is stretched. Then, since the power for the stretching is power whose torque is increased during deceleration by the first transmission mechanism T1, even when a large load is applied to the electric prosthetic leg 1 when moving the electric prosthetic leg 1 forward to ascend stairs, the knee joint mechanism 130 can also be reliably stretched from the bending state.

On the other hand, in order to smoothly ascend stairs, as shown in (E) to (H) of FIG. 21, it is necessary to bend (raise) the knee joint mechanism 130 from the stretched state while a load is applied to a healthy leg. When the knee joint mechanism 130 is bent from the stretched state, large power is not required, but quick action is required.

At this time, the transmission T is set to a second speed-change state where the operation rod 241 is located at the lower position ((C) of FIG. 8). In the second speed-change state, the connection and disconnection unit 212 is in the off state and the connection and disconnection unit 222 is in the on state, and the motor M and the bevel gear mechanism 140 are in the power transmission state via the second transmission mechanism T2.

In this state, when the motor M is rotated in the reverse rotation direction, the power of the motor M is transmitted to the first shaft 181, the second drive gear 185, the second driven gear 186, the connection and disconnection unit 222 of the second connection and disconnection mechanism 220, the second shaft 182, and the bevel gear mechanism 140. With the rotation of the first bevel gear 141 fixed to the below-knee member 110, the second bevel gear 142 fixed to the above-knee member 120 rotates, and thereby the above-knee member 120 rotates about the coupling axis 135 with respect to the below-knee member 110, and knee joint mechanism 130 is bent. Since the power for the bending is power whose torque is reduced during acceleration by the second transmission mechanism T2, the knee joint mechanism 130 can be quickly bent.

FIG. 22 is a diagram showing actions (flat ground walking actions) of the user and the electric prosthetic leg when walking on the flat ground. (A) to (D) of FIG. 22 are diagrams showing a stance phase, (D) of FIG. 22 is a diagram showing a transition phase from a stance to a swing, (E) to (H) of FIG. 22 are diagrams showing a swing phase, and (H) of FIG. 22 is a diagram showing a transition phase from the swing to the stance, and the stance phase.

During walking on the flat ground shown in FIG. 22 and during descending of stairs (stair descending), as shown in (A) to (D) of FIG. 22, in a state where a load is applied to the electric prosthetic leg 1, the transmission T is set to the second speed-change state where the operation rod 241 is positioned at the lower position ((C) of FIG. 8). In the second speed-change state, the connection and disconnection unit 212 is in the off state and the connection and disconnection unit 222 is in the on state, and the motor M and the bevel gear mechanism 140 are in the power transmission state via the second transmission mechanism T2. In the state, when the motor M is in a non-driven state, an external force in the bending direction acting on the electric prosthetic leg 1 is transmitted from the bevel gear mechanism 140 to the motor M via the second transmission mechanism T2, so that frictions of the motor M and the transmission T are utilized to dampen the external force in the bending direction, thus preventing a so-called giving way.

As shown in (D) to (H) of FIG. 22, in a state where a load is applied to the healthy leg, the transmission T is set to the state where the operation rod 241 is positioned at the upper position ((A) of FIG. 8). In this state, the connection and disconnection unit 212 is in the off state and the connection and disconnection unit 222 is in the off state, and the motor M and the bevel gear mechanism 140 are in the power non-transmittable state. Accordingly, the user of the electric prosthetic leg 1 can smoothly swing the leg.

In the enlarging and reducing device 200 of the first embodiment, since the formed angle formed between the below-knee member 110 and the above-knee member 120 can be enlarged and reduced by a rotary mechanism including the bevel gear mechanism 140 but not by an expansion-contraction device such as a spindle mechanism, a length of the electric prosthetic leg 1 in a longitudinal direction (the upper-lower direction) can be reduced. Further, since the rotary mechanism is completed by the bevel gear mechanism 140 in which the first bevel gear 141 and the second bevel gear 142 mesh with each other, complication of the structure can be avoided. Further, since the motor M is disposed at a position overlapping at least a part of the second bevel gear 142 as viewed in the upper-lower direction orthogonal to the rotation axis P2 of the second bevel gear 142, projection of the motor M can be prevented and the electric prosthetic leg 1 can be further reduced in size.

### (Second Embodiment)

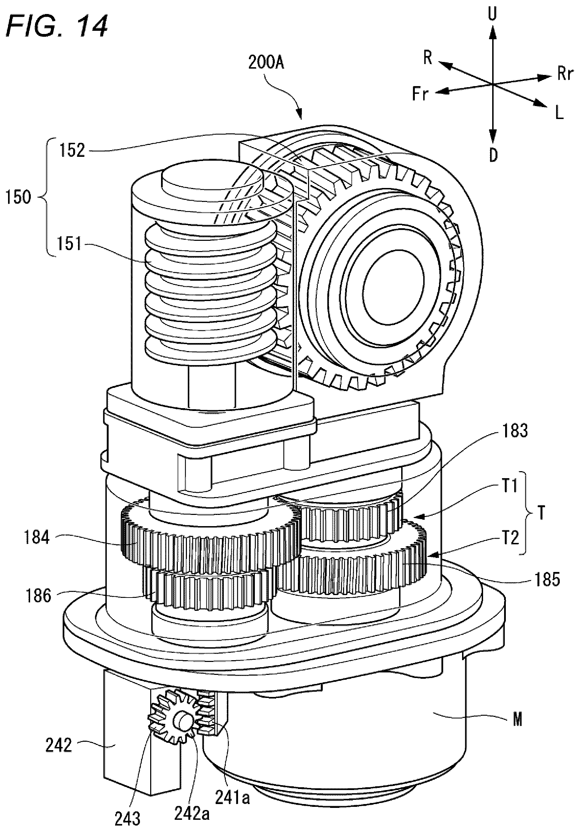

Next, an enlarging and reducing device 200A of a second embodiment will be described with reference to FIGS. 14 to 16.

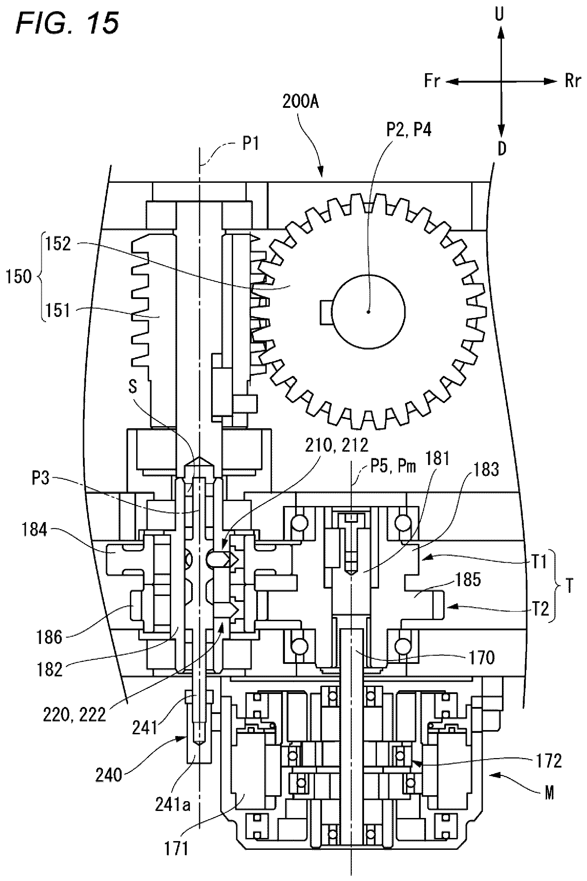

As shown in FIGS. 14 and 15, the enlarging and reducing device 200A includes the motor M that outputs rotational power, the transmission T that transmits the power of the motor M, a worm gear mechanism 150 that can enlarge and reduce the formed angle formed between the below-knee member 110 and the above-knee member 120, and the first connection and disconnection mechanism 210 and the second connection and disconnection mechanism 220 which are provided in the transmission T. Since the configuration other than the worm gear mechanism 150 is the same as that of the enlarging and reducing device 200 of the first embodiment, description thereof will be omitted.

The worm gear mechanism 150 includes a worm gear 151 that is disposed on a side opposite to the motor M with respect to the transmission T on a power transmission path of the motor M and is supported by the below-knee member 110, and a worm wheel 152 that meshes with the worm gear 151 to be capable of transmitting rotation therebetween and is supported by the above-knee member 120. The worm gear 151 and the worm wheel 152 constitute the worm gear mechanism 150 (screw gear mechanism) in which teeth are formed on a cylindrical outer surface thereof. The worm wheel (helical gear) may be replaced with a thin spur gear.

The rotation axis P1 of the worm gear 151 (second shaft 182) and the rotation axis P2 of the worm wheel 152 are disposed to extend in directions orthogonal to each other. In relation to the rotation axis Pm of the motor M, the rotation axis P1 of the worm gear 151 is arranged to extend in a direction parallel to the rotation axis Pm of the motor M, and the rotation axis P2 of the worm wheel 152 is arranged to extend in a direction orthogonal (perpendicular) to the rotation axis Pm of the motor M. The motor M is disposed at a position overlapping at least a part of the worm wheel 152 as viewed in the upper-lower direction orthogonal to the rotation axis P2 of the worm wheel 152.

The worm gear 151 is configured to integrally rotate with the second shaft 182 which is the output element of the transmission T. The worm gear 151 transmits, to the worm wheel 152, the power of the motor M transmitted via the transmission T.

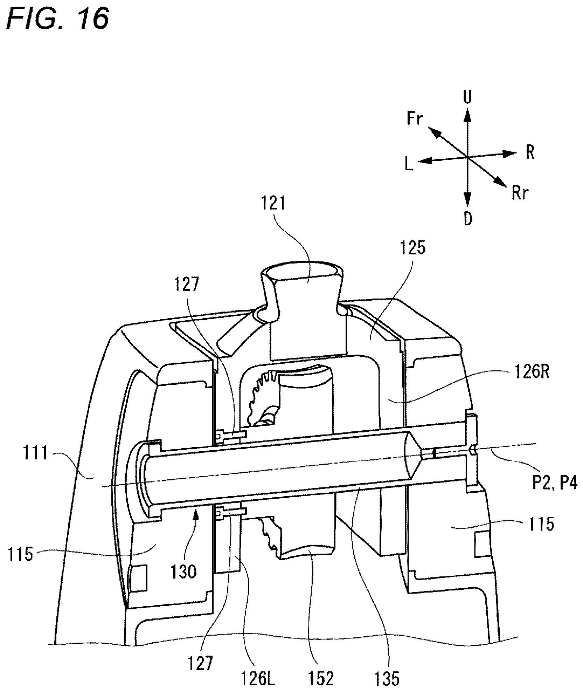

The worm wheel 152 is provided to be rotatable relative to the coupling axis 135 and rotatable integrally with the above-knee member 120. Similar to the second bevel gear 142 of the first embodiment, the worm wheel 152 is provided to be rotatable relative to the coupling axis 135, which is prevented from rotating by the coupling axis support plate 115 of the main frame 111, and to be rotatable integrally with the above-knee member 120. That is, as shown in FIG. 16, the worm wheel 152 is integrally fastened to the left side wall portion 126L by the bolts 127. The side wall portions 126L and 126R are configured to rotate relative to the coupling axis 135 via bearings (not shown). Thus, when worm wheel 152 meshing with worm gear 151 rotates, the above-knee member 120 rotates about the coupling axis 135. Therefore, the formed angle formed between the above-knee member 120 and the below-knee member 110 varies.

In the enlarging and reducing device 200A of the second embodiment, since the formed angle formed between the below-knee member 110 and the above-knee member 120 can be enlarged and reduced by a rotary mechanism including the worm gear mechanism 150 but not by an expansion-contraction device such as a spindle mechanism, a length of the electric prosthetic leg 1 in a longitudinal direction (the upper-lower direction) can be reduced. Further, since the rotary mechanism is completed by the worm gear mechanism 150 in which the worm gear 151 and the worm wheel 152 mesh with each other, complication of the structure can be avoided. Further, since the motor M is disposed at a position overlapping at least a part of the worm wheel 152 as viewed in the upper-lower direction orthogonal to the rotation axis P2 of the worm wheel 152, projection of the motor M can be prevented and the electric prosthetic leg 1 can be further reduced in size.

### (Third Embodiment)

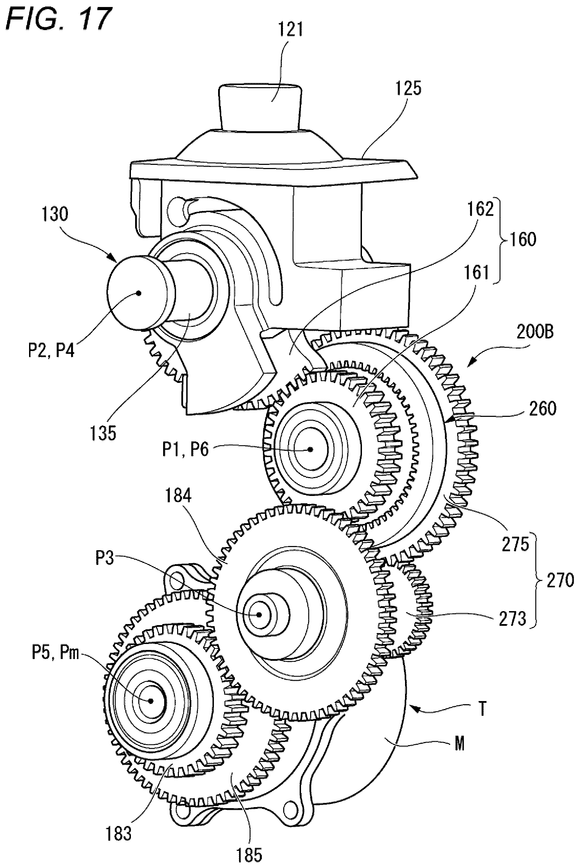

Next, an enlarging and reducing device 200B of a third embodiment will be described with reference to FIGS. 17 to 20.

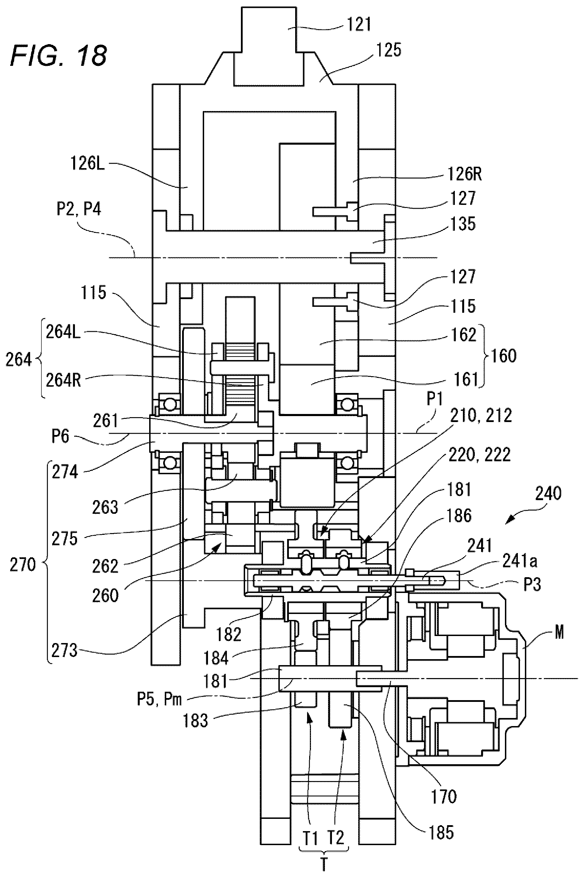

As shown in FIGS. 17 and 18, the enlarging and reducing device 200B includes the motor M that outputs rotational power, the transmission T that transmits the power of the motor M, a spur gear mechanism 160 that can enlarge and reduce the formed angle formed between the below-knee member 110 and the above-knee member 120, an intermediate gear mechanism 270 that is provided between the transmission T and the spur gear mechanism 160, and the first connection and disconnection mechanism 210 and the second connection and disconnection mechanism 220 which are provided in the transmission T.

In the enlarging and reducing device 200B of the third embodiment, the configurations of the motor M and the transmission T are the same as those of the first and second embodiments, but a layout of the motor M and the transmission T is different. That is, in the enlarging and reducing device 200 of the first embodiment and the enlarging and reducing device 200A of the second embodiment, the rotation axis Pm of the motor M, the rotation axis P5 of the first shaft 181 of the transmission T, and the rotation axis P3 of the second shaft 182 are arranged orthogonal to the axis P4 of the coupling axis 135, but in the enlarging and reducing device 200B of the third embodiment, the rotation axis Pm of the motor M, the rotation axis P5 of the first shaft 181 of the transmission T, and the rotation axis P3 of the second shaft 182 are arranged to extend in a direction parallel to the axis P4 of the coupling axis 135.

Specifically, in the enlarging and reducing device 200 of the first embodiment and the enlarging and reducing device 200A of the second embodiment, the rotation axis Pm of the motor M, the rotation axis P5 of the first shaft 181 of the transmission T, and the rotation axis P3 of the second shaft 182 are arranged to extend in the upper-lower direction with respect to the axis P4 of the coupling axis 135 extending in the left-right direction, but in the enlarging and reducing device 200B of the third embodiment, the rotation axis Pm of the motor M, the rotation axis P5 of the first shaft 181 of the transmission T, and the rotation axis P3 of the second shaft 182 are arranged to extend in the left-right direction with respect to the axis P4 of the coupling axis 135 extending in the left-right direction. Since the configurations of the motor M and the transmission T are the same as those in the first and second embodiments, detailed description thereof will be omitted.

The spur gear mechanism 160 includes a pinion gear 161 that is disposed on a side opposite to the motor M with respect to the transmission T on a power transmission path of the motor M and is supported by the below-knee member 110, and a knee axis gear 162 that meshes with the pinion gear 161 to be capable of transmitting rotation therebetween and is supported by the above-knee member 120.

The rotation axis P1 of the pinion gear 161 and the rotation axis P2 of the knee axis gear 162 are arranged to extend in directions parallel to each other. That is, the pinion gear 161 and the knee axis gear 162 are configured to transmit rotation therebetween using a spur gear meshing mechanism. In the present embodiment, the rotation is transmitted by the meshing of two gears, but the rotation may be transmitted by friction between two rollers. That is, the expression "capable of transmitting rotation" may refer to transmission based on the meshing of gears, transmission based on the friction between rollers, or the like.

In relation to the rotation axis Pm of the motor M, the rotation axis P1 of the pinion gear 161 and the rotation axis P2 of the knee axis gear 162 are arranged to extend in a direction parallel to the rotation axis Pm of the motor M.

The pinion gear 161 is configured to integrally rotate with a planetary carrier 264 which is an output element of a planetary gear mechanism 260 to be described later. The pinion gear 161 transmits, to the knee axis gear 162, the power of the motor M transmitted via the transmission T and the intermediate gear mechanism 270. The pinion gear 161 is disposed to fall within an extending-direction length of the coupling axis 135, as viewed in the upper-lower direction orthogonal to the rotation axis P1 of the pinion gear 161. The extending-direction length of the coupling axis 135 is a distance between a left end portion and a right end portion of the coupling axis 135 shown in FIG. 16.

Similar to the second bevel gear 142 of the first embodiment and the worm wheel 152 of the second embodiment, the knee axis gear 162 is provided to be rotatable relative to the coupling axis 135, which is prevented from rotating by the coupling axis support plate 115 of the main frame 111, and to be rotatable integrally with the above-knee member 120. The knee axis gear 162 is a spur gear and has a fan shape in which teeth are only formed partially in the circumferential direction to satisfy the movable range of the second formed angle θ2, and most of the circular spur gear in the circumferential direction is missing. Thus, when the knee axis gear 162 meshing with the pinion gear 161 rotates, the above-knee member 120 rotates about the coupling axis 135. Therefore, the formed angle formed between the above-knee member 120 and the below-knee member 110 varies.

The intermediate gear mechanism 270 includes an intermediate drive gear 273 that rotates integrally with the second shaft 182 that is an output element of the transmission T, an intermediate shaft 274 that has a rotation axis P6 parallel to the rotation axis P3 of the second shaft 182, and an intermediate driven gear 275 that is attached to the intermediate shaft 274 to rotate integrally therewith and meshes with the intermediate drive gear 273. The rotation axis P6 of the intermediate shaft 274 is the same as the rotation axis P1 of the pinion gear 161. The intermediate drive gear 273 and the intermediate driven gear 275 are disposed to fall within the extending-direction length of the coupling axis 135, as viewed in the upper-lower direction orthogonal to the rotation axis P1 of the pinion gear 161.

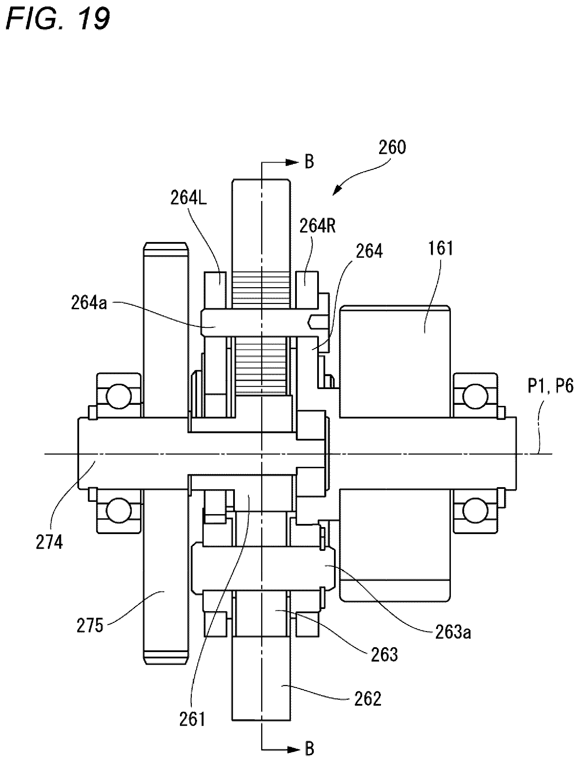

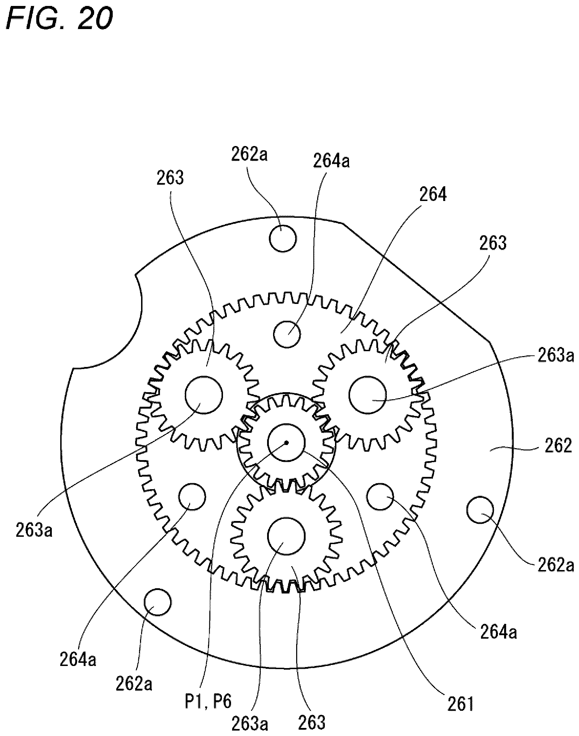

As shown in FIGS. 19 and 20, the planetary gear mechanism 260 is disposed on the rotation axis P6 of the intermediate shaft 274. The planetary gear mechanism 260 includes a sun gear 261 and a ring gear 262 which have the same rotation axis as the intermediate shaft 274, a plurality of planetary gears 263 that mesh with the sun gear 261 and the ring gear 262, and the planetary carrier 264 that supports the plurality of planetary gears 263 in a rotatable and revolvable manner. The three rotary elements including the sun gear 261, the ring gear 262, and the planetary carrier 264 satisfy a collinear relationship in which respective rotation speeds are always aligned on a single straight line in a velocity collinear diagram. The planetary gear mechanism 260 is disposed to fall within the extending-direction length of the coupling axis 135, as viewed in the upper-lower direction orthogonal to the rotation axis P1 of the pinion gear 161.

In the planetary carrier 264, a pair of left and right carrier plates 264L and 264R are coupled by a left-right coupling shaft 264a. The planetary gears 263 are supported between the carrier plates 264L and 264R by pinion rotation shafts 263a in a rotatable and revolvable manner. In the planetary gear mechanism 260, the sun gear 261 is integrally rotatably coupled to the intermediate shaft 274, and the pinion gear 161 is integrally rotatably coupled to the planetary carrier 264. The ring gear 262 is provided with ring fixing holes 262a, and the ring gear 262 is fixed to the main frame 111. Accordingly, the power of the motor M transmitted from the transmission T via the intermediate gear mechanism 270 is input to the sun gear 261 of the planetary gear mechanism 260. Then, in the planetary gear mechanism 260, the rotation of the sun gear 261 is decelerated and transmitted to the planetary carrier 264, and is transmitted to the knee axis gear 162 via the pinion gear 161.

In the enlarging and reducing device 200B of the third embodiment, since the formed angle formed between the below-knee member 110 and the above-knee member 120 can be enlarged and reduced by a rotary mechanism including the planetary gear mechanism 260 but not by an expansion-contraction device such as a spindle mechanism, a length of the electric prosthetic leg 1 in a longitudinal direction (the upper-lower direction) can be reduced. Further, since the rotary mechanism is completed by the planetary gear mechanism 260, the intermediate gear mechanism 270, and the spur gear mechanism 160, complication of the structure can be avoided. Further, since the pinion gear 161, the intermediate drive gear 273, the intermediate driven gear 275, and the planetary gear mechanism 260 are each disposed to fall within the extending-direction length of the coupling axis 135, as viewed in the upper-lower direction orthogonal to the rotation axis P1 of the pinion gear 161, the electric prosthetic leg 1 can be further reduced in size in the extending direction of the coupling axis 135 (the left-right direction of the present embodiment).

Although the various embodiments have been described above with reference to the drawings, it is needless to say that the present invention is not limited to these examples. It is apparent that those skilled in the art can conceive of various modifications and changes within the scope described in the claims, and it is understood that such modifications and changes naturally fall within the technical scope of the present invention. In addition, constituent elements in the embodiment described above may be freely combined without departing from the gist of the present invention.

For example, in the above embodiments, although the prosthetic leg devices (electric prosthetic leg) applied to a knee joint as embodiments of the joint device of the present invention using the connection and disconnection device are described, the present invention is not limited thereto, and may be a prosthetic limb device (electric prosthetic limb) applied to an elbow joint, and the wearer may be an animal other than a human, or a robot. When applied to an elbow joint, the below-knee member 110 in the above embodiment is situated at a distal end side of a wearer with respect to the above-knee member 120, that is, a forearm.

In the present description, at least the following matters are described. In the parentheses, the corresponding constituent elements and the like in the above embodiment are shown, but the present invention is not limited thereto.
(1) Ajoint device (electric prosthetic leg 1) including:
   a first member (below-knee member 110);
   a second member (above-knee member 120);
   a coupling unit (knee joint mechanism 130) configured to couple the first member and the second member such that a formed angle formed between the first member and the second member is variable; and
   an enlarging and reducing device (enlarging and reducing device 200) capable of enlarging and reducing the formed angle formed between the first member and the second member, in which
   the enlarging and reducing device includes a power source (motor M), and a power transmission unit (transmission T) configured to transmit power of the power source,
   the power transmission unit has:
      a first power transmission path (first transmission mechanism T1) for transmitting the power at a first transmission ratio; and
      a second power transmission path (second transmission mechanism T2) for transmitting the power at a second transmission ratio different from the first transmission ratio, and
   the enlarging and reducing device includes:
      a first connection and disconnection mechanism (first connection and disconnection mechanism 210) configured to switch between connection and disconnection of power in the first power transmission path; and
      a second connection and disconnection mechanism (second connection and disconnection mechanism 220) configured to switch between connection and disconnection of power in the second power transmission path.
   According to (1), the coupling unit can be stretched and bent via the power transmission unit that transmits the power of the power source. Since the power transmission unit has two power transmission paths having different transmission ratios, an operation speed and generated power for stretching and bending in the coupling unit can be switched.
(2) The joint device according to (1), in which
   the enlarging and reducing device includes:
   a first rotation member (first bevel gear 141, worm gear 151, and pinion gear 161) that is disposed on a side opposite to the power source with respect to the power transmission unit on a transmission path of the power, and is supported by the first member; and
   a second rotation member (second bevel gear 142, worm wheel 152, and knee axis gear 162) that is disposed to be capable of transmitting rotation with the first rotation member ,and is supported by the second member.
   According to (2), since the enlarging and reducing of the formed angle in the enlarging and reducing device are implemented by rotation transmission between the first rotation member supported by the first member and the second rotation member supported by the second member, the joint device can be reduced in size as compared with a spindle mechanism. The expression "capable of transmitting rotation" may refer to transmission based on meshing of gears, transmission based on friction between rollers, or the like.
(3) The joint device according to (2), in which
   the first rotation member and the second rotation member are disposed such that a first rotation axis (rotation axis P1) that is a rotation axis of the first rotation member and a second rotation axis (rotation axis P2) that is a rotation axis of the second rotation member extend in directions orthogonal to each other.
   According to (3), the rotation can be transmitted using a conical gear meshing mechanism or a conical friction wheel mechanism.
(4) The joint device according to (3), in which
   the first rotation member and the second rotation member constitute a bevel gear mechanism (bevel gear mechanism 140) in which teeth are formed on a conical or truncated conical outer surface.
   According to (4), manufacturing costs of the joint device can be reduced by using a highly versatile mechanism.
(5) The joint device according to (3), in which
   the first rotation member and the second rotation member constitute a screw gear mechanism (worm gear mechanism 150) in which teeth are formed on a cylindrical outer surface.
   According to (5), the manufacturing costs of the joint device can be reduced by using a highly versatile mechanism.
(6) The joint device according to any one of (3) to (5), in which
   the power source is a rotational power source (motor M), and
   a third rotation axis (rotation axis Pm) that is a rotation axis of the rotational power source is arranged to extend parallel to the first rotation axis.
   According to (6), the enlarging and reducing device can be simply configured.
(7) The joint device according to (2), in which
   the first rotation member and the second rotation member are disposed such that a first rotation axis (rotation axis P1) that is a rotation axis of the first rotation member and a second rotation axis (rotation axis P2) that is a rotation axis of the second rotation member extend in directions parallel to each other.
   According to (7), the rotation can be transmitted using a gear meshing mechanism, or a cylindrical friction wheel or grooved friction wheel mechanism.
(8) The joint device according to (7), in which
   the power source is a rotational power source (motor M), and
   a third rotation axis (rotation axis Pm) that is a rotation axis of the rotational power source is arranged to extend in a direction parallel to the first rotation axis and the second rotation axis.
   According to (8), the enlarging and reducing device can be simply configured.
(9) The joint device according to any one of (2) to (8), in which
   the power transmission unit includes:
   a third rotation member (first drive gear 183) disposed on the first power transmission path;
   a fourth rotation member (second drive gear 185) disposed on the second power transmission path, and disposed on a fourth rotation axis (rotation axis P5) that is identical to a rotation axis of the third rotation member;
   a fifth rotation member (first driven gear 184) disposed on the first power transmission path, and disposed to be capable of transmitting rotation with the third rotation member; and
   a sixth rotation member (second driven gear 186) disposed on the second power transmission path, disposed on a fifth rotation axis (rotation axis P3) that is identical to a rotation axis of the fifth rotation member, and disposed to be capable of transmitting rotation with the fourth rotation member.
   According to (9), by arranging the rotation axes of the third rotation member and the fourth rotation member as the same one, and arranging the rotation axes of the fifth rotation member and the sixth rotation member as the same one, the enlarging and reducing device can be reduced in size.
(10) The joint device according to (9), in which
   a first rotation axis (rotation axis P1) that is a rotation axis of the first rotation member and the fifth rotation axis are arranged on a common axis.
   According to (10), by arranging the rotation axes of the first rotation member, the fifth rotation member, and the sixth rotation member as the same one, the enlarging and reducing device can be reduced in size.
(11) The joint device according to (10), in which
   the first rotation member, the fifth rotation member, and the sixth rotation member are disposed on a common axis in this order from one side.
   According to (11), the first power transmission path and the second power transmission path can be disposed close to each other.
(12) The joint device according to any one of (9) to (11), in which
   the power source is a rotational power source (motor M), and
   a third rotation axis (rotation axis Pm) that is a rotation axis of the rotational power source and the fourth rotation axis are arranged on a common axis.
   According to (12), by arranging the rotation axes of the power source, the third rotation member, and the fourth rotation member as the same one, the enlarging and reducing device can be reduced in size.
(13) The joint device according to (12), in which
   the third rotation member, the fourth rotation member, and the rotational power source are disposed on a common axis in this order from one side.
   According to (13), the first power transmission path and the second power transmission path can be disposed close to each other.
(14) The joint device according to any one of (9) to (13), in which
   the power transmission unit includes a seventh rotation member (second shaft 182) connected to the fifth rotation member via the first connection and disconnection mechanism and connected to the sixth rotation member via the second connection and disconnection mechanism.
   According to (14), the first connection and disconnection mechanism and the second connection and disconnection mechanism can be integrated around the seventh rotation member.
(15) The joint device according to (14), in which
   the first connection and disconnection mechanism and the second connection and disconnection mechanism are disposed on the fifth rotation axis.
   According to (15), the first connection and disconnection mechanism and the second connection and disconnection mechanism can be integrated around the fifth rotation axis.
(16) The joint device according to (14) or (15), in which
   the first connection and disconnection mechanism includes:
      a first engagement element (roller 281) disposed between the fifth rotation member and the seventh rotation member; and
      a first operation unit (operation rod 241, pin 283, guide 284, retainer 282) configured to operate the first engagement element to an engagement state and a non-engagement state, and
   the second connection and disconnection mechanism includes:
      a second engagement element (roller 281) disposed between the sixth rotation member and the seventh rotation member; and
      a second operation unit (operation rod 241, pin 283, guide 284, retainer 282) configured to operate the second engagement element to an engagement state and a non-engagement state.
   According to (16), an off state and an on state of the first connection and disconnection mechanism can be switched by the first operation unit, and an off state and an on state of the second connection and disconnection mechanism can be switched by the second operation unit.
(17) The joint device according to (16), in which
   the first operation unit includes:
      a first actuation element (pin 283, guide 284, retainer 282) configured to move the first engagement element; and
      a first operation element (operation rod 241) provided to be capable of operating the first actuation element, and
   the second operation unit includes:
      a second actuation element (pin 283, guide 284, retainer 282) configured to move the second engagement element; and
      a second operation element (operation rod 241) provided to be capable of operating the second actuation element.
   According to (17), the first operation element and the first actuation element constitute the first operation unit, and the second operation element and the second actuation element constitute the second operation unit.
(18) The joint device according to (17), in which
   the first actuation element includes:
      a first advance and retraction element (pin 283) provided to be capable of advancing and retracting along a radial direction with respect to the fifth rotation axis; and
      a first holder (guide 284, retainer 282) that holds the first advance and retraction element to be capable of advancing and retracting,
   the second actuation element includes:
      a second advance and retraction element (pin 283) provided to be capable of advancing and retracting along the radial direction with respect to the fifth rotation axis; and
      a second holder (guide 284, retainer 282) that holds the second advance and retraction element to be capable of advancing and retracting,
   the first operation element is provided to be capable of advancing and retracting along the fifth rotation axis, and an outer periphery of the first operation element is provided to abut on an end portion of the first advance and retraction element on a side of the fifth rotation axis, and
   the second operation element is provided to be capable of advancing and retracting along the fifth rotation axis, and an outer periphery of the second operation element is provided to abut on an end portion of the second advance and retraction element on a side of the fifth rotation axis.
   According to (18), the first advance and retraction element can be advanced and retracted along the radial direction by the first operation element provided to be capable of advancing and retracting along the rotation axis, and the second advance and retraction element can be advanced and retracted along the radial direction by the second operation element provided to be capable of advancing and retracting along the same rotation axis.
(19) The joint device according to (17) or (18), in which
   the seventh rotation member is provided to be hollow to have an internal space (internal space S) extending in a rotation axis direction of the seventh rotation member, and
   the first operation element and the second operation element are integrally formed and disposed to be located in the internal space.
   According to (19), since the first operation element and the second operation element are integrally formed, the number of components can be reduced.
(20) The joint device according to (19), in which
   the enlarging and reducing device includes a drive unit (servo motor 242) configured to drive the first operation element and the second operation element that are integrally formed.
   According to (20), since only one drive unit is required, the enlarging and reducing device can be configured compact.
(21) The joint device according to (20), in which
   the drive unit and the power source are disposed adjacent to each other.
   According to (21), since the drive unit and the power source can be collectively disposed, a driving unit can be made compact.
(22) The joint device according to any one of (9) to (21), in which
   the third rotation member and the fourth rotation member are configured to be capable of integrally rotating.
   According to (22), the number of components can be reduced.
(23) The joint device according to any one of (14) to (21), in which
   the enlarging and reducing device includes an eighth rotation member (intermediate driven gear 275) that is disposed on a sixth rotation axis (rotation axis P6) provided to extend parallel to the fifth rotation axis, and disposed to be capable of transmitting rotation with the seventh rotation member.
   According to (23), the rotation of the seventh rotation member can be transmitted to the eighth rotation member.
(24) The joint device according to (23), in which
   the enlarging and reducing device includes a transmission mechanism (planetary gear mechanism 260) disposed on the sixth rotation axis and disposed to be capable of transmitting rotation with the eighth rotation member.
   According to (24), the rotation of the eighth rotation member can be changed in speed at the transmission mechanism.
(25) The joint device according to (24), in which
   the first rotation member is disposed on the sixth rotation axis and is disposed to be capable of transmitting rotation with the transmission mechanism.
   According to (25), the rotation of the eighth rotation member whose speed has been changed can be transmitted to the first rotation member.
(26) The joint device according to any one of (23) to (25), in which
   the first rotation member and the second rotation member are disposed such that a first rotation axis that is a rotation axis of the first rotation member and a second rotation axis that is a rotation axis of the second rotation member are arranged to extend in directions parallel to each other, and
   the first rotation member is disposed to fall within an extending-direction length of a coupling axis (coupling axis 135) of the coupling unit, as viewed in a direction orthogonal to the first rotation axis.
   According to (26), the joint device can be reduced in size in an extending direction of the coupling axis.
(27) The joint device according to (26), in which
   the eighth rotation member is disposed to fall within the extending-direction length as viewed in a direction orthogonal to the first rotation axis.
   According to (27), the joint device can be reduced in size in the extending direction of the coupling axis.
(28) The joint device according to (26) dependent on (24), in which
   the transmission mechanism is disposed to fall within the extending-direction length as viewed in a direction orthogonal to the first rotation axis.
   According to (28), the joint device can be reduced in size in the extending direction of the coupling axis.
(29) The joint device according to any one of (3) to (6), in which
   the power source is disposed at a position overlapping at least a part of the second rotation member as viewed in a direction orthogonal to the second rotation axis.

According to (29), the joint device can be reduced in size.

The present application is based on a Japanese Patent Application (Patent Application No. 2022-204448) filed on December 21, 2022, and the contents thereof are incorporated herein by reference.

### REFERENCE SIGNS LIST

1 electric prosthetic leg (joint device)
110 below-knee member (first member)
120 above-knee member (second member)
130 knee joint mechanism (coupling unit)
135 coupling axis
140 bevel gear mechanism
141 first bevel gear (first rotation member)
142 second bevel gear (second rotation member)
150 worm gear mechanism
151 worm gear (first rotation member)
152 worm wheel (second rotation member)
160 spur gear mechanism
161 pinion gear (first rotation member)
162 knee axis gear (second rotation member)
182 second shaft (seventh rotation member)
183 first drive gear (third rotation member)
184 first driven gear (fifth rotation member)
185 second drive gear (fourth rotation member)
186 second driven gear (sixth rotation member)
200 enlarging and reducing device
210 first connection and disconnection mechanism
220 second connection and disconnection mechanism
241 operation rod (first operation element, second operation element)
242 servo motor (drive unit)
260 planetary gear mechanism (transmission mechanism)
275 intermediate driven gear (eighth rotation member)
281 roller (first engagement element, second engagement element)
282 retainer (first holder, second holder)
283 pin (first advance and retraction element, second advance and retraction element)
284 guide (First holder, second holder)
M motor (power source, rotational power source)
P1 rotation axis (first rotation axis of first rotation member)
P2 rotation axis (second rotation axis of second rotation member)
P3 rotation axis (fifth rotation axis of each of fifth rotation member and sixth rotation member)
P5 rotation axis (fourth rotation axis of each of third rotation member and fourth rotation member)
P6 rotation axis (sixth rotation axis of eighth rotation member)
Pm rotation axis (third rotation axis of rotational power source)
S internal space
T transmission (power transmission unit)
T1 first transmission mechanism (first power transmission path)
T2 second transmission mechanism (second power transmission path)

## Claims

1. Ajoint device comprising:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the power transmission unit has:
a first power transmission path for transmitting the power at a first transmission ratio; and
a second power transmission path for transmitting the power at a second transmission ratio different from the first transmission ratio, and
the enlarging and reducing device includes:
a first connection and disconnection mechanism configured to switch between connection and disconnection of power in the first power transmission path; and
a second connection and disconnection mechanism configured to switch between connection and disconnection of power in the second power transmission path.

2. The joint device according to claim 1, wherein
the enlarging and reducing device includes:
a first rotation member that is disposed on a side opposite to the power source with respect to the power transmission unit on a transmission path of the power, and is supported by the first member; and
a second rotation member that is disposed to be capable of transmitting rotation with the first rotation member, and is supported by the second member.

3. The joint device according to claim 2, wherein
the first rotation member and the second rotation member are disposed such that a first rotation axis that is a rotation axis of the first rotation member and a second rotation axis that is a rotation axis of the second rotation member extend in directions orthogonal to each other.

4. The joint device according to claim 3, wherein
the first rotation member and the second rotation member constitute a bevel gear mechanism in which teeth are formed on a conical or truncated conical outer surface.

5. The joint device according to claim 3, wherein
the first rotation member and the second rotation member constitute a screw gear mechanism in which teeth are formed on a cylindrical outer surface.

6. The joint device according to any one of claims 3 to 5, wherein
the power source is a rotational power source, and
a third rotation axis that is a rotation axis of the rotational power source is arranged to extend parallel to the first rotation axis.

7. The joint device according to claim 2, wherein
the first rotation member and the second rotation member are disposed such that a first rotation axis that is a rotation axis of the first rotation member and a second rotation axis that is a rotation axis of the second rotation member extend in directions parallel to each other.

8. The joint device according to claim 7, wherein
the power source is a rotational power source, and
a third rotation axis that is a rotation axis of the rotational power source is arranged to extend in a direction parallel to the first rotation axis and the second rotation axis.

9. The joint device according to any one of claims 2 to 8, wherein
the power transmission unit includes:
a third rotation member disposed on the first power transmission path;
a fourth rotation member disposed on the second power transmission path, and disposed on a fourth rotation axis that is identical to a rotation axis of the third rotation member;
a fifth rotation member disposed on the first power transmission path, and disposed to be capable of transmitting rotation with the third rotation member; and
a sixth rotation member disposed on the second power transmission path, disposed on a fifth rotation axis that is identical to a rotation axis of the fifth rotation member, and disposed to be capable of transmitting rotation with the fourth rotation member.

10. The joint device according to claim 9, wherein
a first rotation axis that is a rotation axis of the first rotation member and the fifth rotation axis are arranged on a common axis.

11. The joint device according to claim 10, wherein
the first rotation member, the fifth rotation member, and the sixth rotation member are disposed on a common axis in this order from one side.

12. The joint device according to any one of claims 9 to 11, wherein
the power source is a rotational power source, and
a third rotation axis that is a rotation axis of the rotational power source and the fourth rotation axis are arranged on a common axis.

13. The joint device according to claim 12, wherein
the third rotation member, the fourth rotation member, and the rotational power source are disposed on a common axis in this order from one side.

14. The joint device according to any one of claims 9 to 13, wherein
the power transmission unit includes a seventh rotation member connected to the fifth rotation member via the first connection and disconnection mechanism and connected to the sixth rotation member via the second connection and disconnection mechanism.

15. The joint device according to claim 14, wherein
the first connection and disconnection mechanism and the second connection and disconnection mechanism are disposed on the fifth rotation axis.

16. The joint device according to claim 14 or 15, wherein
the first connection and disconnection mechanism includes:
a first engagement element disposed between the fifth rotation member and the seventh rotation member; and
a first operation unit configured to operate the first engagement element to an engagement state and a non-engagement state, and
the second connection and disconnection mechanism includes:
a second engagement element disposed between the sixth rotation member and the seventh rotation member; and
a second operation unit configured to operate the second engagement element to an engagement state and a non-engagement state.

17. The joint device according to claim 16, wherein
the first operation unit includes:
a first actuation element configured to move the first engagement element; and
a first operation element provided to be capable of operating the first actuation element, and
the second operation unit includes:
a second actuation element configured to move the second engagement element; and
a second operation element provided to be capable of operating the second actuation element.

18. The joint device according to claim 17, wherein
the first actuation element includes:
a first advance and retraction element provided to be capable of advancing and retracting along a radial direction with respect to the fifth rotation axis; and
a first holder that holds the first advance and retraction element to be capable of advancing and retracting,
the second actuation element includes:
a second advance and retraction element provided to be capable of advancing and retracting along the radial direction with respect to the fifth rotation axis; and
a second holder that holds the second advance and retraction element to be capable of advancing and retracting,
the first operation element is provided to be capable of advancing and retracting along the fifth rotation axis, and an outer periphery of the first operation element is provided to abut on an end portion of the first advance and retraction element on a side of the fifth rotation axis, and
the second operation element is provided to be capable of advancing and retracting along the fifth rotation axis, and an outer periphery of the second operation element is provided to abut on an end portion of the second advance and retraction element on a side of the fifth rotation axis.

19. The joint device according to claim 17 or 18, wherein
the seventh rotation member is provided to be hollow to have an internal space extending in a rotation axis direction of the seventh rotation member, and
the first operation element and the second operation element are integrally formed and disposed to be located in the internal space.

20. The joint device according to claim 19, wherein
the enlarging and reducing device includes a drive unit configured to drive the first operation element and the second operation element that are integrally formed.

21. The joint device according to claim 20, wherein
the drive unit and the power source are disposed adjacent to each other.

22. The joint device according to any one of claims 9 to 21, wherein
the third rotation member and the fourth rotation member are configured to be capable of integrally rotating.

23. The joint device according to any one of claims 14 to 21, wherein
the enlarging and reducing device includes an eighth rotation member that is disposed on a sixth rotation axis provided to extend parallel to the fifth rotation axis, and disposed to be capable of transmitting rotation with the seventh rotation member.

24. The joint device according to claim 23, wherein
the enlarging and reducing device includes a transmission mechanism disposed on the sixth rotation axis and disposed to be capable of transmitting rotation with the eighth rotation member.

25. The joint device according to claim 24, wherein
the first rotation member is disposed on the sixth rotation axis and is disposed to be capable of transmitting rotation with the transmission mechanism.

26. The joint device according to any one of claims 23 to 25, wherein
the first rotation member and the second rotation member are disposed such that a first rotation axis that is a rotation axis of the first rotation member and a second rotation axis that is a rotation axis of the second rotation member are arranged to extend in directions parallel to each other, and
the first rotation member is disposed to fall within an extending-direction length of a coupling axis of the coupling unit, as viewed in a direction orthogonal to the first rotation axis.

27. The joint device according to claim 26, wherein
the eighth rotation member is disposed to fall within the extending-direction length as viewed in a direction orthogonal to the first rotation axis.

28. The joint device according to claim 26 dependent on claim 24, wherein
the transmission mechanism is disposed to fall within the extending-direction length as viewed in a direction orthogonal to the first rotation axis.

29. The joint device according to any one of claims 3 to 6, wherein
the power source is disposed at a position overlapping at least a part of the second rotation member as viewed in a direction orthogonal to the second rotation axis.
